# EUROPÄISCHE PATENTSCHRIFT

(11) **EP 0 516 883 B1**
(45) Veröffentlichungstag und Bekanntmachung des Hinweises auf die Patenterteilung: **12.04.1995**
(21) Anmeldenummer: 91121201.7
(22) Anmeldetag: 11.12.1991
(51) Int. Cl.: G02C 5/00, G02C 1/06, G02C 5/12

(54) **Brille, insbesondere Schutzbrille**
Spectacles, particularly protective spectacles
Lunettes, en particulier lunettes de protection

(30) Priorität: 01.06.1991 DE 4118018
(43) Veröffentlichungstag der Anmeldung: 09.12.1992
(73) Patentinhaber: UVEX WINTER OPTIK GmbH, D-90766 Fürth (DE)
(72) Erfinder: Wiedner, Klaus, W-8510 Fürth/Bay. (DE)
(74) Vertreter: Rau, Manfred, Dr. Dipl.-Ing.

(56) Entgegenhaltungen:
- FR-A- 2 475 242
- FR-A- 2 487 084
- FR-A- 2 628 222
- FR-A- 2 652 167
- GB-A- 2 178 864
- US-A- 4 951 322

## Beschreibung

Die Erfindung richtet sich auf eine Brille, insbesondere eine Schutzbrille, mit einem Rahmen, der zwei Sichtscheiben umschließt, wobei zwischen den beiden Sichtscheiben ein Nasensteg an dem Rahmen ausgebildet ist, und wobei ein Halteteil auf den Nasensteg aufschiebbar ist. Eine derartige Brille ist beispielsweise aus FR-A- 2 652 167 bekannt.

Brillen der in Betracht stehenden Art werden als Schutzbrillen, insbesondere beim Sport eingesetzt. Für den Arbeitsschutzbereich sind Brillen bekannt, bei welchen nur eine einzige durchgehende Sichtscheibe vorgesehen ist, welche in einen einstückig gespritzten Rahmen eingesetzt ist. Dementsprechend weist bei diesen Brillen die fertige Brille zwar eine einfache Konstruktion mit einem großen Sichtfeld auf, ist aber den spezifischen anatomischen Gegebenheiten bei dem jeweiligen Benutzer nicht individuell anpaßbar. Weiterhin läßt sich die Sichtscheibe nicht austauschen, so daß z.B. eine spezielle Anpassung der Scheiben an den jeweiligen Einsatzzweck, z.B. durch Auswahl einer entsprechenden Abdunklung, nicht möglich ist. Darüber hinaus könnte durch Schleifstaub oder dergleichen getrübte Scheiben nicht ausgetauscht werden.

Hiervon ausgehend liegt der Erfindung die Aufgabe zugrunde, eine Schutzbrille zu schaffen, die ein hohes Maß an individueller Anpaßbarkeit sowohl an die Anatomie des Benutzers als auch an den jeweiligen Einsatzzweck ermöglicht, die dabei aber gleichwohl eine hohe Schutzwirkung gewährleistet.

Diese Aufgabe wird erfindungsgemäß dadurch gelöst, daß die Sichtscheiben jeweils seitlich an Hinterschneidungen des Rahmens festgelegt sind und im Bereich des Nasensteges durch Aufschieben des Halteteils festgelegt werden.

Es sind an sich bereits zahlreiche Konstruktionen bekanntgeworden, wo die Scheiben durch Haken oder Vorsprünge oder ähnliche Maßnahmen seitlich am Rahmen gehalten sind und im Nasenbereich durch einen lösbaren Verriegelungsmechanismus gehalten werden.

Bei diesen bekannten Brillen handelt es sich aber im wesentlichen um modische Brillen, insbesondere Sonnenbrillen, und weniger um Brillen, bei welchen die Schutzwirkung im Vordergrund steht. Die erfindungsgemäße Ausgestaltung sorgt auch bei Arbeitsschutzbrillen dafür, daß eine solch feste Verankerung der Scheiben erreicht wird, daß diese z.B. der verlangten Beschußfestigkeit gerecht werden.

Darüber hinaus ist von den herkömmlichen Brillen die vorteilhafte weitere Ausgestaltung gemäß der Erfindung nicht bekannt, wonach vorgesehen ist, daß das Halteteil an seiner Unterseite Nasenpads aufweist und demensprechend gleichzeitig als Träger für Nasenpads dient und von der Unterseite her aufgeschoben wird.

Das Halteteil erfüllt dementsprechend eine Doppelfunktion als Träger für die Nasenpads und als Arretierteil für die Scheiben. Die herkömmlichen Arretiermechanismen waren jeweils als gesonderte Teile ausgebildet und verursachten dementsprechend einen zusätzlichen baulichen Aufwand.

Günstigerweise ist weiterhin vorgesehen, daß das Halteteil mit einem Stegabschnitt die Innenränder der Sichtscheiben im Bereich des Nasenstegs übergreift und gegen den Nasensteg festlegt. Hierdurch wird nicht nur eine Festlegung der Scheiben längs eines größeren Bereiches von deren Außenrand erreicht, sondern auch ein sehr ansprechendes Design realisiert.

Mit Vorteil kann weiterhin vorgesehen sein, daß an der Oberseite des Halteteils ein Rastvorsprung angeordnet ist, der in eine korrespondierende Rastausnehmung unter axialer Festlegung an der Oberseite des Rahmens einrastbar ist. Hierdurch wird sichergestellt, daß das Halteteil nach Erreichen seiner Endposition zuverlässig festgelegt ist, was ebenfalls wesentlich zu einer sicheren Festlegung der Scheiben für Arbeitsschutzanwendungen beiträgt.

Gemäß einer weiteren vorteilhaften Ausgestaltung der Erfindung weisen die Sichtscheiben an ihrer Oberseite sich aus der Scheibenebene etwa rechtwinklig heraus nach hinten erstreckende, im eingesetzten Zustand die Oberseite des Rahmens übergreifende Ansätze auf. Hierdurch wird einerseits eine sichere Abdeckung des Bereichs an der Rahmenoberseite gegen das Eindringen von Licht und Fremdkörper erreicht, andererseits werden die Scheiben auf diese Weise in Richtung nach unten fixiert. Letztlich wird so auch eine sehr flüssige Linienführung erreicht, welche zu einem ansprechenden Design führt.

In diesem Sinne kann weiterhin vorgesehen sein, daß die Ansätze an den Scheiben im Nasenstegbereich mit einem dort am Rahmen vorgesehenen, die Rasteinrichtung tragenden Rahmenteil fluchten, so daß bei eingesetzen Scheiben eine durchgehende Brillenoberseite entsteht.

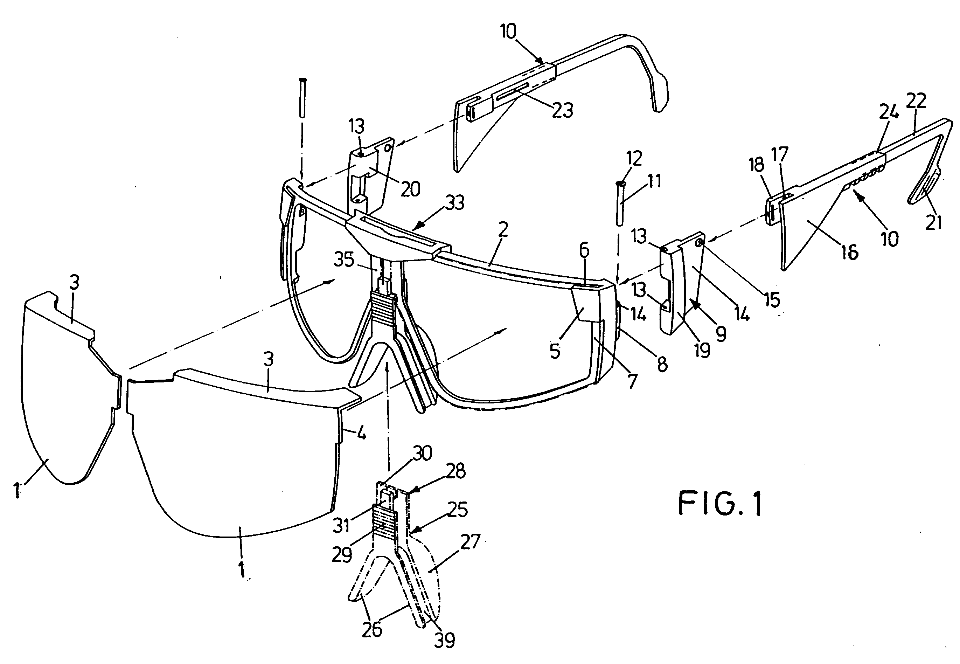

Nachfolgend wird die Erfindung anhand eines bevorzugten Ausführungsbeispieles in Verbindung mit der Zeichnung näher erläutert. Dabei zeigen
- Fig. 1: eine perspektivische Explosionsdarstellung einer erfindungsgemäßen Brille,

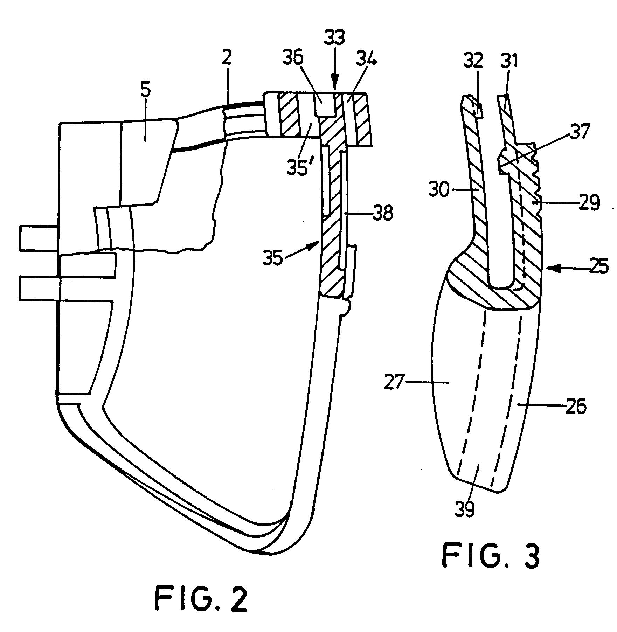

- Fig. 2: einen Schnitt durch den Nasenstegbereich und
- Fig. 3: einen Schnitt durch das auf den Nasenstegbereich aufzusetzende Halteteil.

Eine in der Zeichnung dargestellte Brille umfaßt zwei Scheiben 1 und einen einstückig aus Kunststoff gespritzten Rahmen 2.

An der Oberseite der Scheiben 1 ist jeweils ein Ansatz 3 vorgesehen, welcher sich aus der Ebene der Scheiben 1 senkrecht heraus auf das Gesicht des Benutzers zuerstreckt, wobei der Innenrand jedes Ansatzes 3 der Gesichtsform des Benutzers anatomisch angepaßt ist.

Unterhalb der Ansätze 3 weisen die Scheiben 1 in Verlängerung der Scheibenebene seitliche Vorsprünge 4 auf, welche hinter Ansätzen 5 des Rahmens 2 in einer Hinterschneidung 6 zu liegen kommen, wenn die Scheiben 1 von oben auf den Rahmen 2 aufgesetzt werden.

An der Rückseite der Ansätze 5 sind an dem im Gebrauchszustand vertikalen Rahmenabschnitt 7 Scharnierösen 8 vorgesehen, auf welche ein Lagerteil 9 für einen Bügel 10 die Scharnieransätze 8 umgreifend aufsetzbar ist, wobei ein Scharnierlagerbolzen 11 mit verdicktem oberen Ende 12 in an sich bekannter Weise durch die Scharnierbohrungen 13 des Lagerteils 9 einsetzbar ist, wobei der Bolzen 11 gleichzeitig auch eine Bohrung 14 in dem Scharnieransatz 8 durchsetzt.

Das Lagerteil 9 weist im Anschluß an den Scharnierbereich eine Platte 14 auf, welche an ihrem hinteren oberen Ende eine Lagerbohrung 15 aufweist.

Die Bügel 10 weisen eine seitliche Schutzplatte 16 auf und an deren Innenseite einen Lagerzapfen 17, der in die Lagerbohrung 15 einrastbar ist, wobei ein sich parallel zur Lagerplatte 16 erstreckender Ansatz 18 mit einer an sich bekannten Inklinationsrastung dafür sorgt, daß der Plattenansatz 9 gabelförmig zwischen der Platte 16 und dem Ansatz 18 des Bügels 10 zu liegen kommt. Die Außenseite der Schutzplatte 16 fluchtet mit der Außenseite 19 des Scharnierlagerteils 9 und die Außenseite des Ansatzes 18 fluchtet mit der Innenseite 20 des Scharnierlagerteils 9.

Ein Ohrteil 21 des Bügels 10 weist einen linearen Abschnitt 22 mit wenigstens einem in der Zeichnung nicht dargestellten Rastvorsprung auf, der mit Rastausnehmungen, die im ebenfalls im einzelnen nicht dargestellt sind, in einem seitlichen Schlitz 23 beim Hineinstecken des Abschnittes 22 in eine Ausnehmung 24 des Bügels 10 zur Längenverstellung des Ohrteils 21 zusammenwirkt.

Ein in der Zeichnung in der Mitte unten gestrichtelt und in der Mitte oben durchgezogen im aufgesetzten Zustand dargestelltes Halteteils 25 weist zwei Ansätze 26 auf, die etwa V-förmig angeordnet sind, und an deren Rückseite Nasenpads angeordnet sind.

An der Oberseite der Ansätze 26 ist ein etwa U-förmiges Rastteil 28 vorgesehen, welches eine vordere Platte 29 und eine hintere Platte 30 umfaßt. Beim Aufschieben des Halteteils 25 auf den Rahmen 2 übergreift die vordere Platte 29 die Vorderseite der eingesetzten Scheiben 1 und die hintere Platte 30 übergreift die Rückseite des Nasenstegs 35.

An der Oberseite der vorderen Platte 29 ist ein Führungszapfen 31 und an der Innenseite der hinteren Platte 30 ein Rastvorsprung 32 angeordnet, wobei in einem an der Oberseite des Rahmens 2 befestigten Arretierteil 33 eine Führungsausnehmung 34 für den Führungsansatz 31 und eine Führungsausnehmung 35 für die Platte 30 ausgebildet ist. Die Führungsausnehmung 35'weist eine Hinterschneidung 36 auf, in die der Rastvorsprung 32 definiert einrastet, so daß absolut sichergestellt ist, daß die Scheiben 1 sich nicht unbeabsichtigt lösen können, sondern nur bei einem manuellen Entrasten des Rastvorsprungs 32 das Halteteil 25 zum Scheibenwechseln entfernt werden kann.

Ein Vorsprung 37 an der Innenseite der Platte 29 greift in eine korrespondierende Ausnehmung 38 an der Vorderseite des Arretierteils 33 ein und sorgt so ebenfalls für eine definierte Führung und Festlegung.

Die die Nasenpads 27 tragenden Ansätze 26 weisen Nuten 39 auf, die den Außenrand an der Unterseite des Rahmens 2 und der Scheiben 1 umgreifen und auf diese Weise für eine zusätzliche Festlegung sorgen.

## Patentansprüche

1. Brille, insbesondere Schutzbrille, mit einem Rahmen, der zwei Sichtscheiben umschließt, wobei zwischen den beiden Sichtscheiben ein Nasensteg an dem Rahmen ausgebildet ist, und wobei ein Halteteil auf den Nasensteg aufschiebbar ist, dadurch gekennzeichnet, daß die Sichtscheiben (1) jeweils seitlich an Hinterschneidungen (6) des Rahmens (2) festgelegt sind und im Bereich des Nasensteges durch das Aufschieben des Halteteils (25) festgelegt werden.

2. Brille nach Anspruch 1, dadurch gekennzeichnet, daß das Halteteil (25) an seiner Unterseite Nasenpads (27) aufweist.

3. Brille nach Anspruch 1, dadurch gekennzeichnet, daß das Halteteil (25) mit einem Stegabschnitt (Platten 29, 30) die Innenränder der Sichtscheiben (1) im Bereich des Nasenstegs (35) übergreift.

4. Brille nach Anspruch 1, dadurch gekennzeichnet, daß das Halteteil (25) im Bereich der Nasenpads (27) Nuten (39) aufweist, die im aufgesetzten Zustand den Außenrand des Rahmens (2) unterhalb des Nasenstegs (35) übergreifen und den Rand der Sichtscheiben (1) zusätzlich festlegen.

5. Brille nach Anspruch 1, dadurch gekennzeichnet, daß an der Oberseite des Halteteils (25) ein Rastvorsprung (32) angeordnet ist, der in eine korrespondierende Rastausnehmung (36) unter axialer Festlegung an der Oberseite des Rahmens (2) einrastbar ist.

6. Brille nach Anspruch 1, dadurch gekennzeichnet, daß die Sichtscheiben (1) an ihrer Oberseite sich aus der Scheibenebene etwa rechtwinklig heraus nach hinten erstreckende, im eingesetzten Zustand die Oberseite des Rahmens (2) übergreifende Ansätze (3) aufweisen.

7. Brille nach Anspruch 6, dadurch gekennzeichnet, daß die Ansätze (3) an den Scheiben (1) im Nasenstegbereich (35) mit einem dort vorgesehenen, die Rasteinrichtung tragenden Arretierteil (33) fluchten.

## Claims

1. Glasses, in particular safety glasses, with a frame encircling two sight pieces, a bridge being formed on the frame between the two sight pieces, and a holder being placeable on to the bridge, characterized in that the sight pieces (1) are each laterally fixed to rear recesses (6) of the frame (2) and arrested in the vicinity of the bridge by the holder (25) being pushed on.

2. Glasses according to claim 1, characterized in that the holder (25) has nose pads (27) on its underside.

3. Glasses according to claim 1, characterized in that a rib section (plates 29, 30) of the holder (25) overlaps the inner edges of the sight pieces (1) in the vicinity of the bridge (35).

4. Glasses according to claim 1, characterized in that in the vicinity of the nose pads (27), the holder (25) is provided with grooves (39) which, the holder (25) being placed, overlap the outer edge of the frame (2) below the bridge (35) and additionally arrest the edges of the sight pieces (1).

5. Glasses according to claim 1, characterized in that a locking protrusion (32) is disposed on the upper side of the holder (25), which locking protrusion (32) is lockingly engageable with a corresponding locking recess (36) on the upper side of the frame (2) and is thus axially fixed.

6. Glasses according to claim 1, characterized in that the sight pieces (1) have appendages (3) on their upper side which extend approximately rearwards at right angles to the plane of the sight pieces and which overlap the upper side of the frame in the inserted position.

7. Glasses according to claim 6, characterized in that in the vicinity of the bridge (35), the appendages (3) of the sight pieces (1) are in alignment with a locking member (33) provided in the vicinity of the bridge (35) and supporting the locking device.

## Revendications

1. Lunettes, notamment lunettes protectrices, avec une monture qui entoure deux verres, une arcade étant formée entre les deux verres sur la monture et une pièce de serrage étant plaçable sur l'arcade, caractérisées en ce que les verres (1) sont arrêtés latéralement sur des contre-dépouilles (6) de la monture (2) et qu'ils sont fixés dans le domaine de l'arcade par la mise-en-place d'une pièce de serrage (25).

2. Lunettes selon la revendication 1, caractérisées en ce que la pièce de serrage (25) présente des coussins (27) pour le nez sur son côté inférieur.

3. Lunettes selon la revendication 1, caractérisées en ce qu'une partie en nervure (plaques 29, 30) de la pièce de serrage (25) recouvre les bords intérieurs des verres (1) dans le domaine de l'arcade (35).

4. Lunettes selon la revendication 1, caractérisées en ce que dans le domaine des coussins (37) pour le nez, la pièce de serrage (25) présente des rainures (39, qui, en état de mise-en-place de la pièce de serrage (25), recouvrent le bord extérieur de la monture (2) au-dessous de l'arcade (35) et arrêtent le bord des verres (1) additionellement.

5. Lunettes selon la revendication 1, caractérisées en ce qu'une partie en saillie (32) à encliqueter est arrangée sur le côté supérieur de la pièce de serrage (25), saillie qui peut être encliquetée dans un évidement correspondant d'encliquetage (36) sur le côté supérieur de la monture (2) tout en étant fixée axialement.

6. Lunettes selon la revendication 1, caractérisées en ce que sur leur côté supérieur, les verres (1) présentent des rallonges (3) qui s'étendent à-peu-près perpendiculairement vers l'arrière et recouvrent le côté supérieur de la monture quand les verres ont été mis en place.

7. Lunettes selon la revendication 6, caractérisées en ce que dans le domaine de l'arcade (35), les rallonges (3) sur les verres (1) sont en alignement avec une pièce d'arrêt (33) qui est pourvue dans ce domaine et comporte le dipositif d'encliquetage.
